# EUROPEAN PATENT APPLICATION

(11) **EP 1 038 956 A1**
(43) Date of publication of application: **27.09.2000**
(21) Application number: 99105846.2
(22) Date of filing: 23.03.1999
(51) Int. Cl.: C12N 15/10, C12N 15/12, C12N 15/62, C07K 14/435

(54) **Detection, cloning and sequencing of polypeptides which drive the subcellular localization of proteins**

(71) Applicant: EUROPÄISCHES LABORATORIUM FÜR MOLEKULARBIOLOGIE (EMBL), D-69117 Heidelberg (DE)
(72) Inventor: The designation of the inventor has not yet been filed
(74) Representative: Böhm, Brigitte, Dipl.-Chem. Dr.

(57) **Abstract**

The present invention concern a process for the detection, cloning and/or sequencing of polypeptides or parts thereof, which drive the subcellular localization of a protein containing such polypeptide or part thereof, **characterized** in that the process comprises the following steps:
(a) constructing an expression library of random nucleic acids ligated to a reporter gene and contained in a vector molecule,
(b) transfecting a plurality of host cells with the library,
(c) screening for the subcellular localization of the expression product of the nucleic acid in the host cells via detection of a signal produced by the reporter gene,
(d) cloning such cells where the reporter gene signal is detected in a certain subcellular localization, and
(e) cloning and optionally sequencing the nucleic acid insert which encodes the polypeptide or part thereof.

Polypeptides, driving the intracellular localization can be used to construct fusion proteins with predetermined intracellular localization.

## Description

The present invention relates to a process for the detection, cloning and/or sequencing of polypeptides or parts thereof, which drive the subcellular localization of a protein containing such polypeptide or part thereof, a process for the identification and/or production of a protein that is localized in a given subcellular localization, and a process for directing the subcellular localization of a nucleic acid expression product.

One of the most conspicuous features of the eukaryotic cell is its high degree of compartmentalization. Chromatin, nuclear matrix, nuclear membrane, Golgi apparatus, endoplasmatic reticulum, the endo- and exocytic compartments, the actin and microtubule cytoskeletons, mitochondria, the centrosome and the cell membrane are just some of the major subcellular organelles/compartments which have been defined by standard cytological analysis. Moreover, most of these compartments can be further subdivided into well differentiated regions or structures having a cytological and molecular identity of their own, thus resulting in the large number of subcellular domains which characterize the eukaryotic cell.

The physiological relevance of such compartmentalization is paramount. Every major cellular activity can be assigned to one or more well defined subcellular compartments. As a matter of fact, the intricate regulatory networks which operate within eukaryotic cells greatly rely on the differential subcellular localization of the molecules involved. The close relationship between subcellular localization and function is such that in most instances determining the preferential subcellular localization of a protein provides one of the best clues as to its putative function.

The molecular basis of specific subcellular localizations is not yet well understood. In some cases the localized protein contains a functional domain which drives its targeting either directly or through interaction with other previously localized members of the target structure. Well known examples of this first case are the nuclear localization signals which are recognized by the nuclear pore complex and translocated into the nucleus or the combination of a polybasic domain and a C-terminal CAAX motif, which leads to the post-translational modification of the protein and its membrane targeting. The second case includes kinesins and MAPs the cytoskeletal, spindle or centrosomal localization of which is achieved by virtue of their interaction with microtubuli. Finally, it is also conceivable that other mechanisms, e.g. different rates of import, export and degradation, could result in steady-states which may account for the preferential subcellular localization of proteins which do not contain any bona fide subcellular localization signal of their own.

In view of the diversity of mechanisms which may account for the subcellular localization of a given protein, it was an object of the present invention to provide a possibility to detect signals which either directly or indirectly drive subcellular localization. Such signals are usually polypeptides or parts thereof that are present in proteins. The knowledge of such polypeptides can be useful for a plurality of assays or applications.

The object underlying the invention was accomplished by the provided process for the detection, cloning and/or sequencing of polypeptides or parts thereof, which drive the subcellular localization of a protein containing such polypeptide, wherein said process comprises:
(a) constructing an expression library of random nucleic acids ligated to a reporter gene and contained in a vector molecule,
(b) transfecting a plurality of host cells with the library,
(c) screening for the subcellular localization of the expression product of the nucleic acid in the host cells via detection of a signal produced by the reporter gene,
(d) cloning such cells where the reporter gene signal is detected in a certain subcellular localization of interest, and
(e) cloning and optionally sequencing the nucleic acid insert which encodes the polypeptide or part thereof.

The above process allows for the detection of polypeptides or parts thereof, which drive the targeting of a protein to a particular subcellular location or structure, said process being completely independent of the function, organization and length of the respective protein containing such polypeptide.

Depending on the length of the random nucleic acids the process of the invention also allows the detection of complete or nearly complete proteins, that due to the presence of a polypeptide driving the localization are transfered to a certain intracellular location after expression. According to the invention random nucleic acids are produced from the genome of an organism. Either genomic DNA or cDNA may be used to generate the random nucleic acids that are ligated to a reporter molecule and inserted in vector molecules to construct the expression library of feature (a). Random nucleic acid molecules that are produced by subjecting the DNA of interest to restriction cleavage are only detected in the location of interest if they contain at least such a portion of said polypeptide which ensures that the subcellular localization driving function is retained. Other constructs might also lead to expression of a fusion protein displaying the reporter gene signal albeit not in the localization of interest.

Out-of-frame insertions are generally not expressed in the process according to the invention.

It is irrelevant whether the random nucleic acid and reporter gene are ligated before being inserted in a vector molecule together, or whether a vector containing the reporter gene is constructed into which a random nucleic acid can easily be ligated in an appropriate location next to the reporter gene. It is generally preferred to produce a fusion gene product, with the reporter gene located at the C-terminus. If the reporter gene is located at the N-terminal end, it will always be expressed thereby increasing background. Nevertheless, this might be the only way to detect some proteins which will not get localized if the fusion is made in the other direction. Theoretically, it is also possible that the reporter gene product is located in between nucleic acid expression products, as long as both the reporter gene and/or the polypeptide or part thereof retain their function.

The thus obtained vector molecules containing the random nucleic acid and reporter gene are transfected into a plurality of host cells which are then subjected to conditions allowing for the expression of the vector insert, whereupon screening for the subcellular localization of the expression product of the nucleic acid in the host cells takes place via detection of a signal produced by the reporter gene. Cells which show the reporter gene signal in a subcellular localization of interest are selected and subcloned, whereupon the DNA sequence insert can be cloned and optionally sequenced, said nucleic acid insert encoding the polypeptide or part thereof.

In a preferred embodiment of the present invention, a cDNA is used to create the random nucleic acids, or an expression library made of a cDNA is used.

In a further preferred embodiment of the invention, a library, either genomic or cDNA, from a mammalian organism or yeast or C. elegans or C. laevis is used. This preferred embodiment is not intended to limit the invention, since DNA from any organism may be used to detect specific polypeptide or parts thereof which drive the subcellular localization in the respective organism.

In one embodiment of the invention, a homologous system of nucleic acid for the creation of the expression library and host cell for the transfection is used. The homologous expression system is meant to identify a system where host cells are used that belong to the same species from which the nucleic acid was obtained.

In another embodiment of the invention, a heterologous system of nucleic acid library and cells for the transfection is used. In such a case, the host cell does not belong to the same species as the nucleic acid that is to be expressed therein.

An example for a heterologous system would be the use of a Drosophila DNA for the generation of the expression library and of mammalian or yeast cells as host cells to be transfected with the vector molecule.

For the process according to the invention, standard procedures can be used which are known to the man in the art. Cloning of cells can be done either manually, picking up the cells and replating them for as many times as required to isolate one clone, or by serial dilution. Also, a fax sorter may be used which separates individual cells expressing a specific marker.

In a preferred embodiment of the invention, a reporter gene leading to a visually detectable signal upon expression is used.

Although principally other reporter genes are suitable, too, the visually detectable expression product is most easily detectable. Especially preferred reporter genes are genes coding for GFP (green fluorescent protein), or GFP derivatives like for example BFP (blue fluorescent protein), luciferase, YFP (yellow fluorescent protein), or CFP (cyan fluorescent protein). These derivatives are described in Pepperkok et al., Current Biology 9: 269 - 272 (1999) and references quoted therein.

The process according to the invention makes it possible to establish a system allowing to screen a huge number of nucleic acid molecules for the presence of a sequence encoding a polypeptide or part thereof capable of driving the subcellular localization of a protein containing such polypeptide.

Therefore, a further subject of the present invention is a process for identifying and/or producing a protein that is localized in a given subcellular location, such process comprising the above process according to the invention as well as the cloning of a nucleic acid coding for a polypeptide epitope driving the localization in this given subcellular localization, and the use of said cloned nucleic acid to detect longer DNA sequences coding for a protein containing such polypeptide epitope. This detection can be conducted by standard molecular biology techniques, for example by hybridizing the nucleic acid to a genomic or cDNA library from a certain species and detecting homologous sequences encoding a protein, by RT-PCR or by comparing the obtained nucleic acid sequence with databases containing a huge number of DNA sequences. Such databases contain sequences coding for known proteins as well as sequences which are postulated to be coding for a protein, which, however, has either not been identified yet or the function of which is still unknown.

As soon as, by the above process for the identification of a protein containing the polypeptide, the respective nucleic acid coding for such protein is obtained or the sequence thereof is known, said nucleic acid can be expressed in an expression system, producing said protein containing a polypeptide or used in any other way including formation of mutants etc.

A still further subject of the present invention is a process for directing the subcellular localization of a nucleic acid expression product. Said process comprises detecting a polypeptide or part thereof driving the localization of a protein containing such polypeptide according to the above process of the invention, and obtaining the nucleotide sequence encoding such polypeptide or part thereof, wherein further the nucleic acid coding for the polypeptide or part thereof is fused to a nucleic acid coding for a protein to be expressed, and the fusion product is expressed.

In a preferred embodiment of the present invention, the nucleic acid for the polypeptide or part thereof driving the localization and a reporter gene are fused with the nucleic acid coding for a protein to be expressed. By such fusion of a polypeptide and a reporter gene with a nucleic acid, the actual expression of the protein to be expressed at the localization of interest can be monitored.

For this purpose, it is preferred that a reporter gene is used the expression product of which is visually detectable.

It is a further preferred but in no way obligatory embodiment of the invention that the fusion product of the protein to be expressed and polypeptide or part thereof and/or reporter gene contains a proteolytic cleavage site between the protein to be expressed and the polypeptide and/or the reporter gene product. According to this preferred embodiment, it is possible to obtain the pure protein to be expressed in a given localization by cleaving off the part directing the localization and optionally also the part enabling the monitoring of the expression and localization.

To this end, it might be useful to also express a corresponding proteolytic enzyme and direct it to the same subcellular localization by means of the process of the invention.

The following examples along with the accompanying figures are intended to further elucidate the invention:
Fig. 1 A - D shows a schematic representation of the process steps performed in Example 1.
Fig. 2 shows examples of subcellular localization of a reporter protein.

### Example 1

### Creating the Td2 fragment in EGFP-N1

The vector pEGFP-N1 (CLONTECH Laboratories, Inc., 1020 East Meadow Circle, Palo Alto, CA 94303-4230, USA) was modified by PCR using the following primers:

Oligo A (5′-CATGTTGGCGGCCGCGGTACCGTCGA-3′)*
Oligo B (5′-GCCCGGGCGTGAGCAAGGGCGAG-3′).

Oligo A contains an ATG with a good Kozak and a Srfl site. The ATG is out of frame with the GFP coding sequence.

PCR was carried out with Expand High Fidelity PCR System (Boehringer Mannheim GmbH, Sandhofer Strasse 116, D-68305 Mannheim, Germany) as indicated in the following protocol:

| Step | Temperature | Time |
|---|---|---|
| 1 | 94°C | 3 min |
| 2 | 42°C | 1 min |
| 3 | 68°C | 4 min (slope 13°C/sg) |
| 4 | 94°C | 1 min |
| 5 | 42°C | 1 min |
| 6 | 68°C | 4 min (goes to 4, 7 cycles) |
| 7 | 68°C | 7 min |
| 8 | 4°C | pause |

The PCR product was purified using the PCR Quiaquick Purification System (QIAGEN GmbH, Max-Valmer-Strasse 4, 40724 Hilden, Germany) and ligated with Rapid Ligation Kit (Boehringer Mannheim GmbH, supra). The ligated vector was used to transfect Epicurian Coli XL1-Blue (Stratagene, 11011 North Torrey Pines Road, La Jolla, CA 92097) by heat shock. After transfection, the cells were plated out in LB-Agar supplemented with 30 µg/ml kanamycin and incubated for 16 hours at 37°C. The DNA of some of the resulting colonies was isolated by minipreps and analyzed with restriction enzymes to confirm that it corresponded to the expected modified vector.

From this modified vector, the Td2 fragment was purified and subcloned into the Notl site of vectors of the pQE30 series (previously modified to contain this site). As expected, only Td2 fragments cloned into the pQE30 Notl resulted in cells expressing GFP. After this check, the Td2 fragment was cloned back into the NotI site of pEGFP-N1 NotI. This is a derivative of pEGFP-N1 into which a NotI site was introduced in the polylinker. The final vector is pEGFPTd2.

### Modification of the pQE30 series

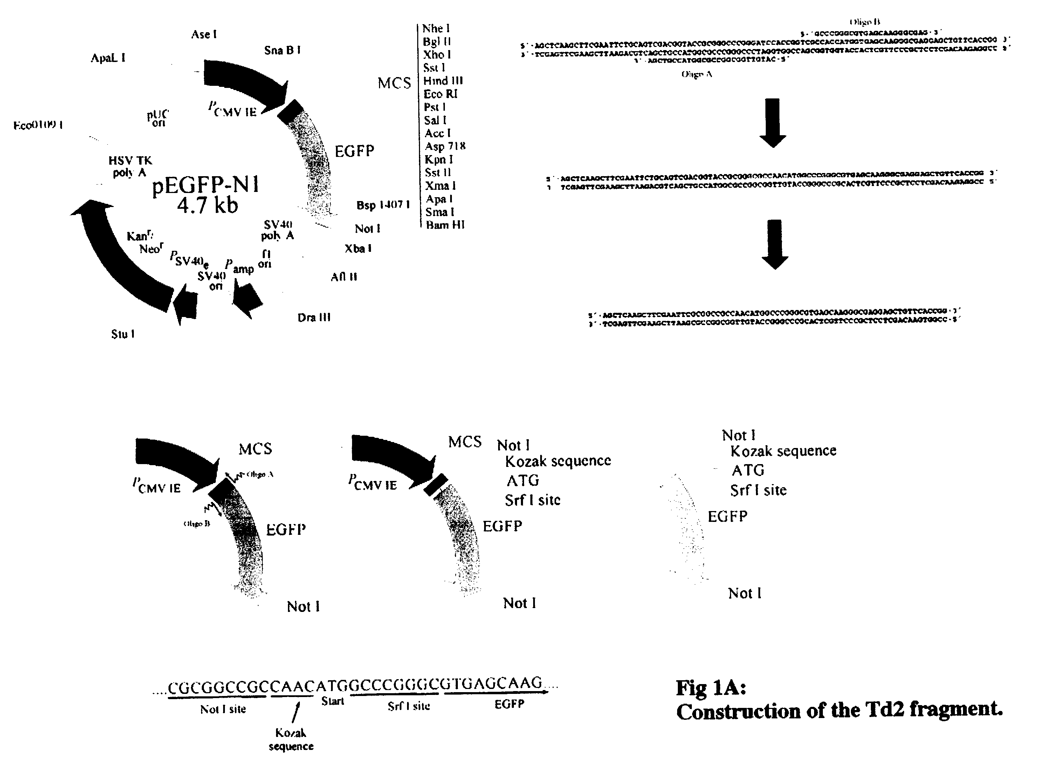

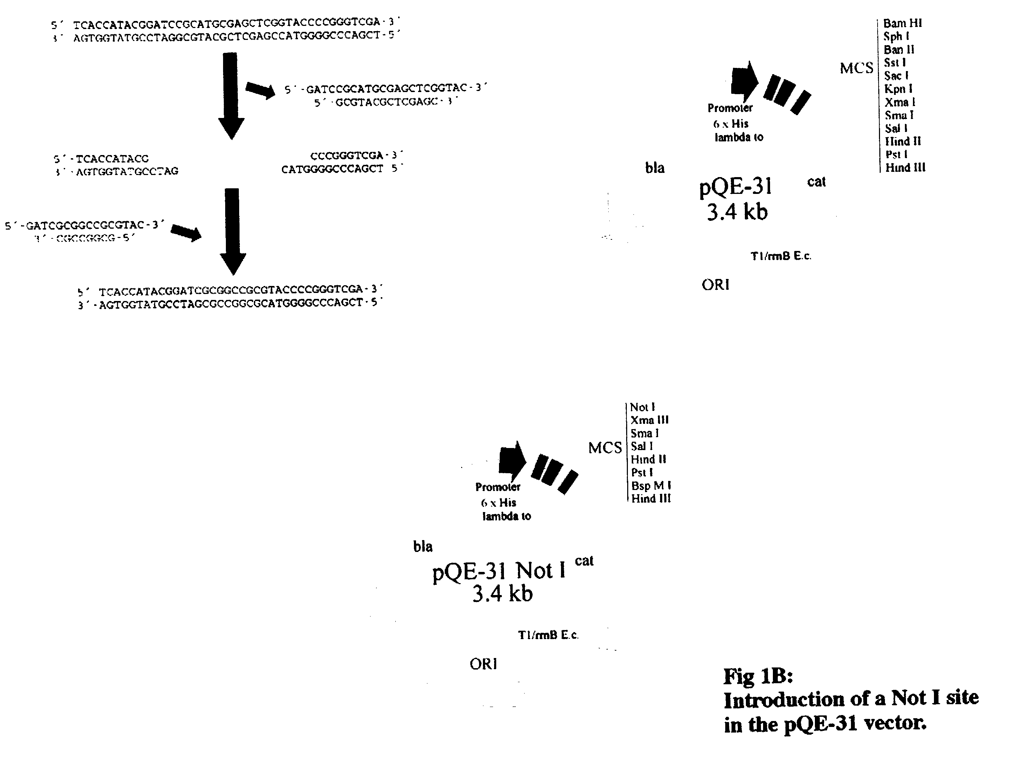

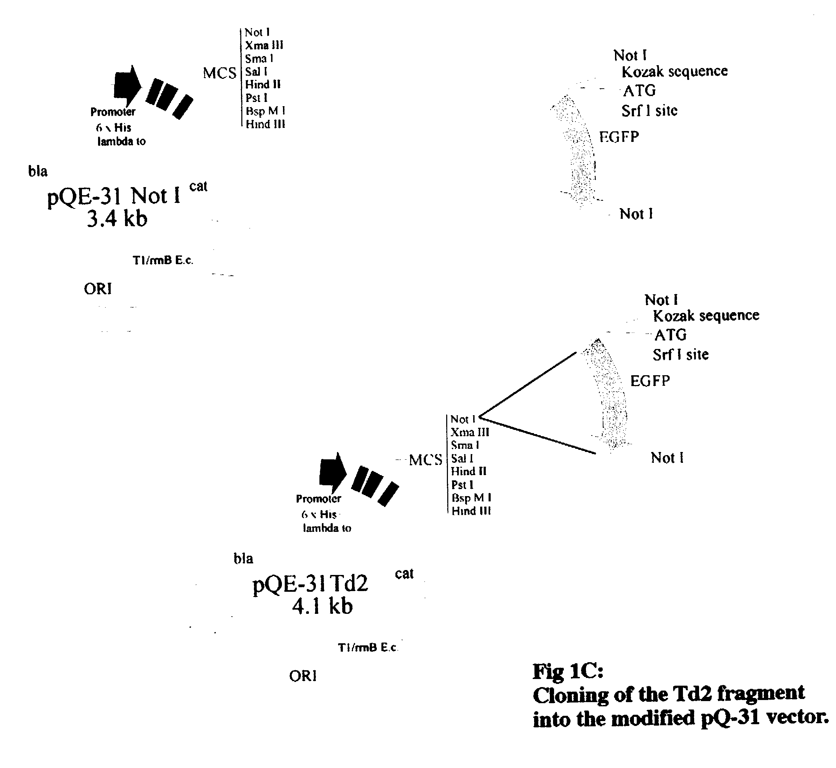

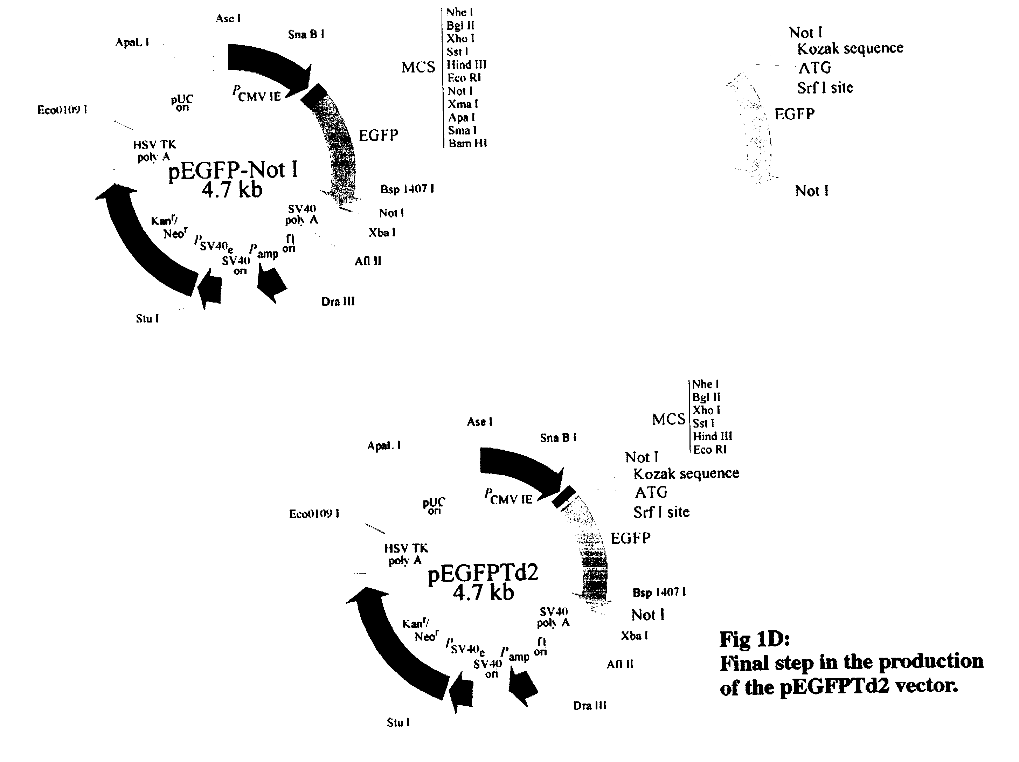

These were modified introducing between the Bam HI and Kpnl sites an adapter containing a Notl site. The adapter was made by annealing the following oligos: 
Not 1-1b (5′-GATCGCGGCCGCGTAC-3′)
Not 1-8 (5′-GCGGCCGC-3′).
 Fig. 1 A - D shows the procedures of Example 1 schematically.

### Example 2

### Construction of the "epitope-trap" library

Drosophila gDNA was cut to completion with Alul and Haelll (Boehringer Mannheim, supra), purified with QIAEXII Gel Extraction Kit, run in agarose gel for further size selection, purified again with QIAEXII Gel Extraction Kit and cloned into the Srfl of pEGFPTd2. Ligated DNA was used to transform E. coli XL1-Blue MR (Stratagene, supra). A small fraction of the cells was plated in LB medium containing 30 µg/ml kanamycin. The resulting clones were isolated and their DNA was purified and analyzed to determine the size of the average insert. The results were about 420,000 clones, approx. 6,700 (1.6%) of which had no insert, the estimated average length of the inserts being around 490 bp.

The remaining cells were plated out over a sterile nylon filter laid on a 24x24 plate containing LB supplemented with kanamycin, incubated at 30°C for 24 hours, replicated into another filter and reincubated for 4 hours at 37°C. The DNA was then obtained using Plasmid Maxi kit by QIAGEN.

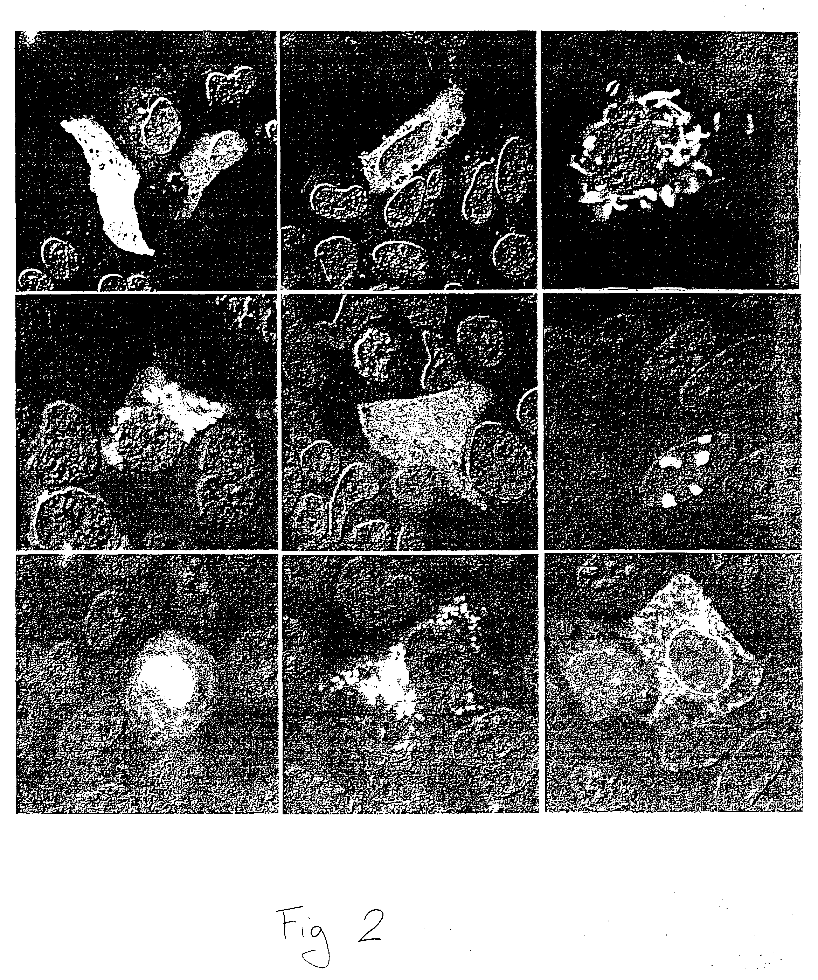

Cells were transfected with a library prepared as described above. Ten hours after transfection, cells were fixed with methanol, and observed under the microscope. The reporter protein used in this example (GFP) can be observed as a bright white. In each of the examples shown in Fig. 2, the reporter is localized in different components within the cells. Observations were made with a Leica TCS confocal microscope system (Leica, Germany).

## Claims

1. Process for the detection, cloning and/or sequencing of polypeptides or parts thereof, which drive the subcellular localization of a protein containing such polypeptide or part thereof,
**characterized** in that the process comprises the following steps:
(a) constructing an expression library of random nucleic acids ligated to a reporter gene and contained in a vector molecule,
(b) transfecting a plurality of host cells with the library,
(c) screening for the subcellular localization of the expression product of the nucleic acid in the host cells via detection of a signal produced by the reporter gene,
(d) cloning such cells where the reporter gene signal is detected in a certain subcellular localization, and
(e) cloning and optionally sequencing the nucleic acid insert which encodes the polypeptide or part thereof.

2. Process according to claim 1,
**characterized** in that a cDNA or cDNA fragments are used as random nucleic acids.

3. Process according to claim 1 or 2,
**characterized** in that a eukaryotic or a yeast library is used.

4. Process according to anyone of claims 1 to 3,
**characterized** in that a homologous system of library and cells for the transfection is used.

5. Process according to anyone of claims 1 to 3,
**characterized** in that a heterologous system of library and cells for the transfection is used.

6. Process according to claim 5,
**characterized** in that a Drosophila library is used to transfect mammalian or yeast cells.

7. Process according to anyone of claims 1 to 6,
**characterized** in that a reporter gene leading to a visually detectable signal upon expression is used.

8. Process according to claim 7,
**characterized** in that nucleic acids coding for GFP, BFP, luciferase or YFP are used as reporter gene.

9. Process according to anyone of claims 1 to 8,
**characterized** in that the vector contains an inducible promoter driving the expression of random nucleic acid and marker gene.

10. Process for the identification and/or production of a protein that is localized in a given subcellular localization,
**characterized** in that a nucleic acid coding for a polypeptide or part thereof driving the localization in said given subcellular localization is cloned according to claims 1 to 9 end the nucleic acid is used to detect DNA sequences coding for a protein containing such polypeptide or part thereof.

11. Process according to claim 10,
**characterized** in that for the production of the protein the nucleic acid is expressed in an expression system.

12. Process for directing the subcellular localization of a nucleic acid expression product,
**characterized** in that a polypeptide driving the localization of a protein containing such polypeptide or part thereof is detected, its nucleic acid sequence is obtained by a process according to anyone of claims 1 to 8, the nucleic acid coding for the polypeptide or part thereof is fused to a nucleic acid coding for a protein to be expressed, and the fusion product is expressed.

13. Process according to claim 12,
**characterized** in that a nucleic acid coding for the polypeptide or part thereof and a reporter gene is fused to the nucleic acid coding for a protein to be expressed.

14. Process according to claim 12,
**characterized** in that a reporter gene the expression product of which is visually detectable is used.

15. Process according to anyone of claims 12 to 14,
**characterized** in that the fusion product contains a proteolytic cleavage site between the protein to be expressed and the polypeptide or part thereof and/or reporter gene product.
